# EUROPEAN PATENT SPECIFICATION

(11) **EP 4 037 749 B1**
(45) Date of publication and mention of the grant of the patent: **28.05.2025**
(21) Application number: 19782980.7
(22) Date of filing: 01.10.2019
(51) Int. Cl.: A61M 25/01, A61M 25/06

(54) **INTRODUCER SHEATH**
EINFÜHRUNGSHÜLSE
GAINE D'INTRODUCTION

(43) Date of publication of application: 10.08.2022
(73) Proprietor: Clearstream Technologies Limited, Enniscorthy, County Wexford (IE)
(72) Inventor: LIVINGSTON, Jean, Enniscorthy, County Wexford (IE); KAVANAGH, Michaella, Enniscorthy, County Wexford (IE); CAHILL, Lisa, Enniscorthy, County Wexford (IE); REDMOND, Jack, Enniscorthy, County Wexford (IE)
(74) Representative: Hoffmann Eitle
(86) International application number: PCT/EP2019/076558
(87) International publication number: WO 2021/063489

(56) References cited:
- WO-A1-2016/089232
- WO-A1-2016/094068
- JP-A- H0 847 538

## Description

### Technical Field

The present disclosure relates to an introducer sheath for insertion into the body of a patient through an access site of the body.

### Background

An introducer sheath can be used to introduce catheters or other devices safely into a blood vessel through an access site to perform intravascular procedures. The access site may be a brachial, femoral or any other suitable access site.

When performing intravascular procedures, physicians may need to reposition the introducer sheath. However, this may result in unintentional sheath withdrawal which can lead to a lengthened procedure with potentially greater complications such as regaining access.

Current introducer sheaths known in the art only allow the physician to see the access site visually and the introducer sheath tip under fluoroscopy. For example, JPH0847538 and WO 2016/094068 A1 both disclose a sheath with a radiopaque marker disposed at the distal end. These devices do not allow the physician to conveniently and accurately determine the location of the access site under fluoroscopy, which may result in the above-noted issues.

In view of the above, there is a need for an improved introducer sheath which allows a physician to conveniently and reliably locate the access site during intravascular procedures.

JP H08 47538 A discloses a sliding catheter having a catheter pipe, a sliding pipe which is freely axially movably inserted into the catheter pipe and a cylindrical balloon film which is joined at its opening end to the front end of the sliding pipe and is joined at its second opening end to the front end of the catheter pipe. A marker foil consisting of an X-ray impermeable material is attached near at least the front end of the sliding pipe.

WO 2016/094068 A1 discloses a medical device delivery system. The delivery system includes a catheter having a proximal end, a distal end, and a sheath. A bifurcated gripping member is disposed on the proximal end of the catheter and the bifurcated gripping member includes a first gripping prong and a second gripping prong. A deployable prosthesis is disposed on the distal end of the catheter.

### Brief Description of the Drawings

To enable better understanding of the present disclosure, and to show how the same may be carried into effect, reference will now be made, by way of example only, to the accompanying drawings, in which:
FIG. 1 shows a side view of one embodiment of an introducer sheath according to the present disclosure;
FIG. 2 shows the introducer sheath of FIG. 1 in a first configuration;
FIG. 3A shows the introducer sheath of FIG. 1 in a second configuration; and
FIG. 3B shows the introducer sheath of FIG. 1 in the second configuration while a catheter is inserted through the introducer sheath into an access site of the body of a patient.

### Detailed Description

In one aspect of the present disclosure, there is provided an introducer sheath for insertion into the body of a patient through an access site of the body. The introducer sheath comprises a longitudinal sheath member for insertion into the access site to carry out intravascular work. The introducer sheath further comprises a radiopaque marker element arranged over the outer surface of the longitudinal sheath member. The radiopaque marker element is moveable along at least part of the longitudinal length of the longitudinal sheath member from a first position, at which the radiopaque marker element is not in contact with the access site, to a second position, at which the radiopaque marker element abuts the access site such that insertion of the radiopaque marker element into the access site is prevented.

Throughout this disclosure, the term 'introducer sheath' may refer to a device which enables a catheter or other intravascular device to be inserted into a patient's body.

Throughout this disclosure, the term 'access site' may refer to any point on a human or animal body through which access to a blood vessel or other vessel may be gained from outside of the body. For example, the access site may be an incision in the skin of the body.

Throughout this disclosure, the term 'longitudinal sheath member' may refer to a hollow elongated object which is suitable for insertion into a vessel of a human or animal body. The longitudinal sheath member is configured to allow for insertion of another object therethrough, such as a catheter or other elongated device.

Throughout this disclosure, the term 'intravascular work' may refer to any procedure which is carried out within a vessel or vessels of a human or animal body, such as, for example, percutaneous transluminal angioplasty.

Throughout this disclosure, the term 'radiopaque' may refer to the property of a substance being opaque to X-Rays or similar radiation. Any radiopaque material/object referred to herein is radiopaque to such an extent that a practitioner may easily identify the material/object during fluoroscopy. Examples of radiopaque materials that may be used include barium sulfate, bismuth compounds, zirconium oxide, lead, gold, silver, stainless steel, platinum, iridium, tungsten, or tantalum. In certain embodiments, any of the radiopaque materials/objects referred to herein have a radiodensity of at least 1000 Hounsfield Units (HU), optionally at least 5000 Hounsfield Units (HU), further optionally at least 10 000 Hounsfield Units (HU), even further optionally at least 20 000 Hounsfield Units (HU).

Throughout this disclosure, the term 'arranged over' may refer to a configuration where the radiopaque marker element completely or only partially surrounds the longitudinal sheath member. The radiopaque marker element may be fully in contact, partially in contact or not in contact with the longitudinal sheath member.

The introducer sheath is configured such that once the longitudinal sheath member is inserted into the access site, the radiopaque marker element is moveable along at least part of the longitudinal length of the longitudinal sheath member from the first position, at which the radiopaque marker element is not in contact with the access site, to the second position, at which the radiopaque marker element abuts the access site such that insertion of the radiopaque marker element into the access site is prevented.

The introducer sheath may further comprise a second radiopaque marker element fixedly disposed at a distal section of the longitudinal sheath member.

The second radiopaque marker element may be disposed at a distal end of the longitudinal sheath member.

The introducer sheath may comprise a biasing element configured to bias the radiopaque marker element to the second position.

Throughout this disclosure, the term 'biasing element' may refer to an element which upon deformation, exerts a restoring force on an object so as to cause that object to move in a particular direction or force the object into a particular position.

The biasing element may be configured such that, in the relaxed state of the biasing element, the radiopaque marker element is aligned with the distal end of the longitudinal sheath member.

The biasing element may be a spring.

The introducer sheath may further comprise a sheath hub disposed at the proximal end of the longitudinal sheath member, and wherein the spring is disposed between the sheath hub and the radiopaque marker element.

The ends of the spring may be fixedly attached to the sheath hub and the radiopaque marker element, respectively.

The spring may be made from Nitinol.

The radiopaque marker element may comprise a first radiopaque part and a second non-radiopaque part.

The first radiopaque part may be disposed at a distal section of the radiopaque marker element. In certain embodiments, the first radiopaque part is disposed at the distal of the radiopaque marker element.

The radiopaque marker element may consist of a radiopaque material.

The radiopaque marker element may be tapered along a longitudinal length thereof parallel to the longitudinal axis of the longitudinal sheath member.

The radiopaque marker element may be elongate. In certain embodiments, the radiopaque marker element may be tapered along a longitudinal length thereof.

The radiopaque marker element may have a distal section which is wider than a proximal section thereof. The radiopaque marker element may have a distal end which is wider than a proximal end thereof.

The radiopaque marker element may be configured to encompass the access site.

The radiopaque marker element may touch the outer surface of the longitudinal sheath member.

The radiopaque marker element may be movable along the longitudinal length of the longitudinal sheath member by sliding the radiopaque marker element relative to the longitudinal sheath member.

The introducer sheath may be configured such that when the depth of insertion of the longitudinal sheath member is changed, the radiopaque marker element maintains abutting contact with the access site such that insertion of the radiopaque marker element into the access site is prevented.

In a second aspect of the present disclosure not according to the presently claimed invention, there is provided a method of inserting an introducer sheath into an access site of the body of a patient. The method may comprise inserting a longitudinal sheath member into the access site, the longitudinal sheath member being comprised within the introducer sheath. The introducer sheath may have a radiopaque marker element arranged over the outer surface of the longitudinal sheath member. The method may further comprise moving the radiopaque marker element from a first position, at which the radiopaque marker element is not in contact with the access site, to a second position, at which the radiopaque marker element abuts the access site such that insertion of the radiopaque marker element into the access site is prevented.

The introducer sheath may further comprise a biasing element. The biasing element may urge the radiopaque marker element from the first position to the second position.

The method may further comprise changing the depth of insertion of the longitudinal sheath member whilst maintaining abutting contact between the radiopaque marker element and the access site such that insertion of the radiopaque marker element into the access site is prevented.

In further implementations of the method, the introducer sheath further comprises a sheath hub disposed at the proximal end of the longitudinal sheath member and a spring, disposed between the sheath hub and the radiopaque marker element. The spring may be configured to bias the radiopaque marker towards the distal end of the longitudinal sheath member. The spring may be tensioned by placing the radiopaque marker element at the second position.

In a further aspect of the present disclosure there is provided a system comprising an introducer sheath as described throughout this disclosure and a catheter. The catheter comprises a third radiopaque marker element at a distal section of the catheter.

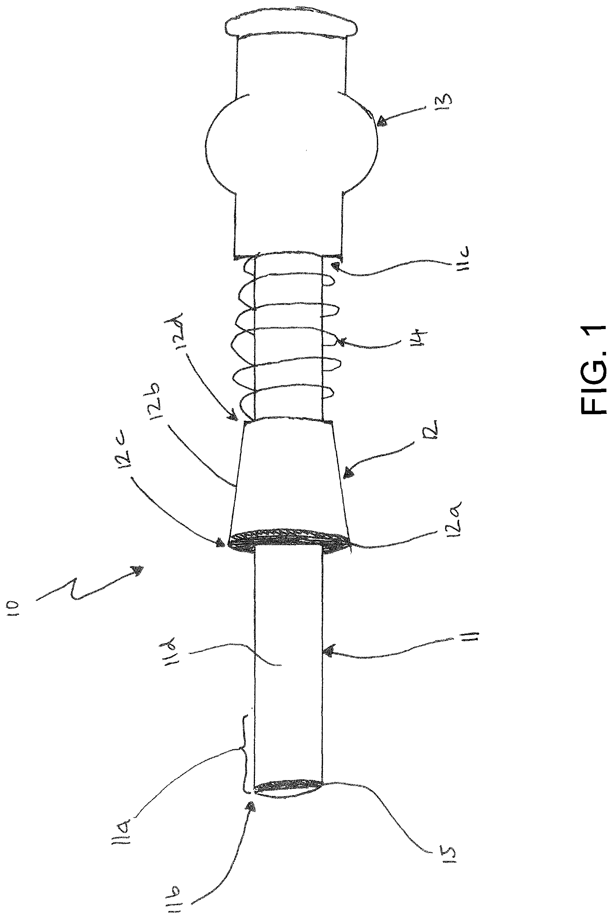

FIG. 1 shows a side view of an introducer sheath 10. The introducer sheath 10 is suitable for insertion into the body of a patient through an access site of the body, to allow introduction of catheters or other devices into the body of the patient to perform intravascular procedures. The access site may be a brachial or femoral access site, for example.

The introducer sheath 10 comprises a longitudinal sheath member 11. The longitudinal sheath member 11 has a distal section 11a, a distal end 11b and a proximal end 11c. The longitudinal sheath member 11 is a tube comprising a lumen which allows a catheter or other intravascular device to be disposed therethrough (see below).

When the introducer sheath 10 is introduced into the body of a patient, the longitudinal sheath member 11 will enter the patient's body through the access site and may be the only part of the introducer sheath 10 which enters the body of the patient.

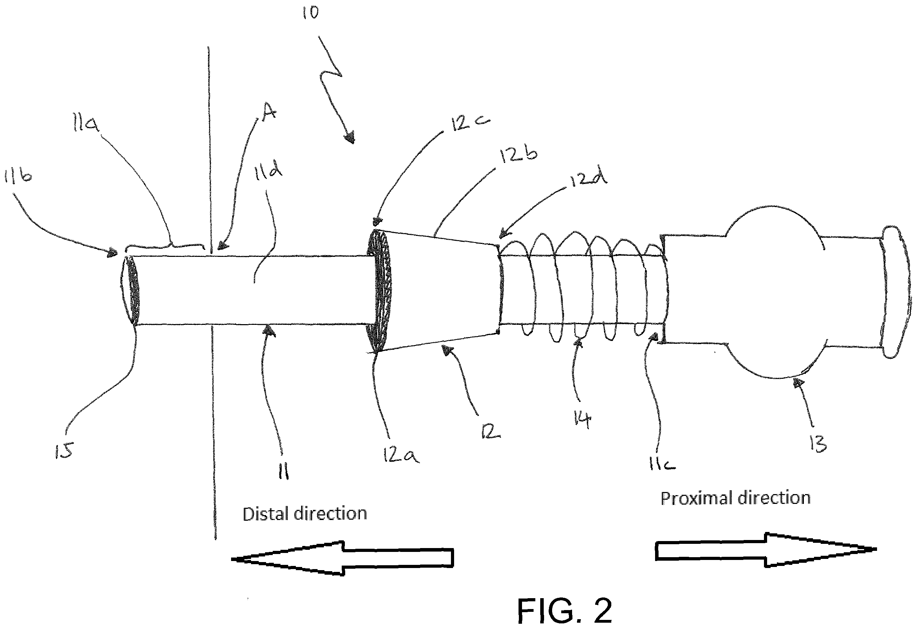

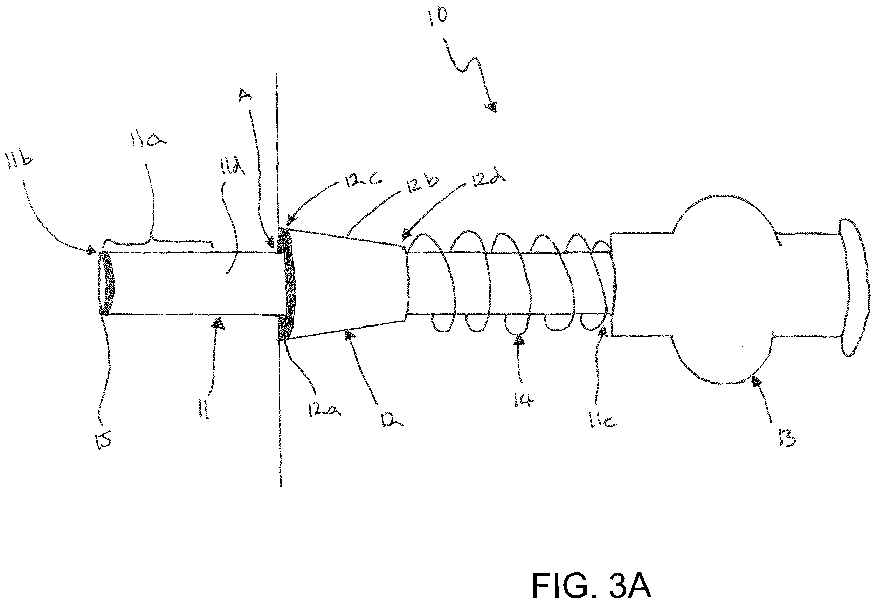

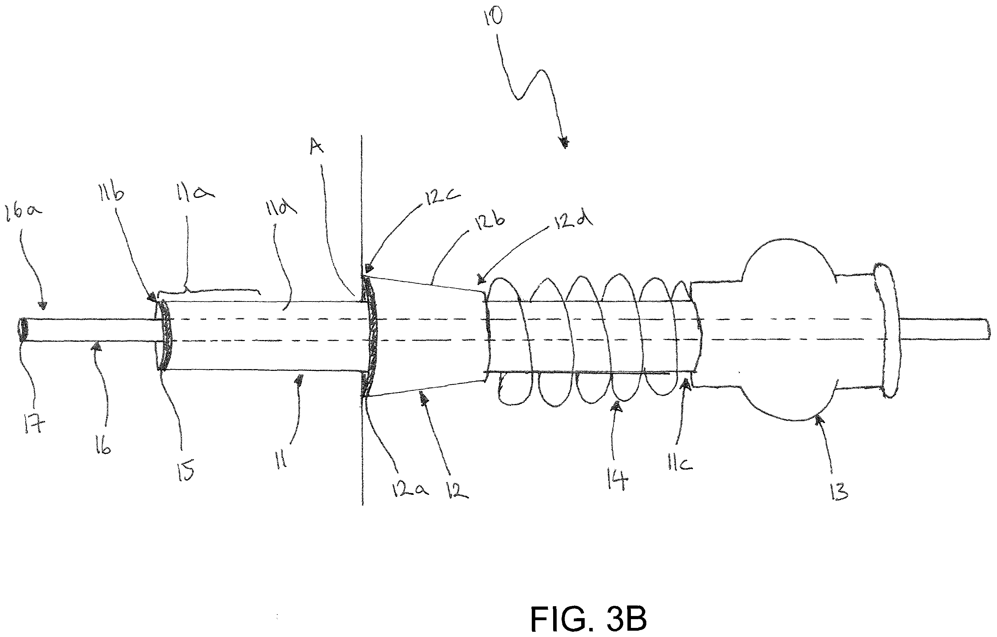

The introducer sheath 10 further comprises a radiopaque marker element 12 arranged over an outer surface 11d of the longitudinal sheath member 11. The radiopaque marker element 12 is moveable along the longitudinal length of the longitudinal sheath member 11. When the introducer sheath 10 is inserted into the body of a patient through an access site, the radiopaque marker element 12 can be moved from a first position, in which the radiopaque marker element 12 is not in contact with the access site, to a second position, in which the radiopaque marker element 12 abuts the access site such that insertion of the radiopaque marker element into the access site is prevented (as described in more detail in FIGS. 2 and 3).

Intravascular procedures are usually carried out under fluoroscopy which allows a physician to see the devices inside the vessel of a patient. When the radiopaque marker 12 is in the second position and abutting the access site, the physician can conveniently and accurately see the location of the access site under fluoroscopy whilst carrying out the intravascular procedure.

As the radiopaque marker element 12 is arranged over the outer surface 11d of the longitudinal sheath member 11, the physician can easily move the radiopaque marker element 12 to a position abutting the access site to give an accurate indication of the location of the access site without losing the radiopaque marker or requiring the use of adhesives. Furthermore, when repositioning the introducer sheath 10, the radiopaque marker 12 can be conveniently repositioned to abut the access site and there is no danger of the radiopaque marker element 12 falling off or being lost.

Therefore, the introducer sheath 10 may allow a physician to conveniently and reliably locate the access site during intravascular procedures.

The longitudinal sheath member 11 further comprises a second radiopaque marker element 15. The second radiopaque marker element 15 is disposed at the distal section 11a of the longitudinal sheath member 11.

The second radiopaque marker element 15 may allow the physician to visualise the distal section 11a of the longitudinal sheath member 11 under fluoroscopy and therefore conveniently determine the distance between the radiopaque marker element 12 disposed at the access site and the second radiopaque marker element 15 disposed at the distal section 11a of the longitudinal sheath member 11 inside the body of the patient, while intravascular procedures are carried out.

This may allow a physician to conveniently and accurately determine the depth of penetration of the introducer sheath 10 into the body so as to minimise accidental sheath withdrawal during intravascular procedures.

In FIG. 1, the second radiopaque marker element 15 is disposed at the distal end 11b which corresponds to the distal-most part of the longitudinal sheath member 11. This may give the most accurate indication of the depth of penetration of the longitudinal sheath member into the body of the patient.

The radiopaque marker element 12 has a distal section 12c and a proximal section 12d. The radiopaque marker element 12 further defines a lumen so that it may be arranged around the outer surface 11d of the longitudinal sheath member 11 so as to touch the outer surface 11d of the longitudinal sheath member 11. The radiopaque marker element 12 is moveable along the longitudinal length of the longitudinal sheath member 11 by sliding the radiopaque marker element 12 relative to the longitudinal sheath member 11.

In FIG. 1, the radiopaque marker element 12 encircles the longitudinal sheath member 11, so that the radiopaque marker element 12 cannot be removed from the longitudinal sheath member 11 unless the radiopaque marker element 12 is slid distally along the longitudinal sheath member 11 past the distal end 11b of the longitudinal sheath member 11.

The radiopaque marker element 12 comprises a first radiopaque part 12a and a second non-radiopaque part 12b. The first radiopaque part 12a is disposed at the distal section 12c of the radiopaque marker element 12, so as to give a more accurate indication of the location of the access site. In the embodiment of FIG. 1, the second radiopaque marker is disposed at the distal-most end of the radiopaque marker element. The second non-radiopaque part 12b is disposed towards the proximal section 12d of the radiopaque marker element.

The first radiopaque part 12a may be composed of any radiopaque material such as a platinum iridium compound, for example. The second non-radiopaque part 12b may be made from a polyether block amide such as PEBAX^{®}.

The radiopaque marker element 12 is tapered along the axis of its lumen (i.e. along the longitudinal length of the sheath member 11) so that the distal section 12c of the radiopaque marker element 12 is wider than the proximal section 12d of the radiopaque marker element 12. The tapering allows for easier holding and placement of the radiopaque marker element 12 at the access site.

The introducer sheath 10 may include a spring 14 connected to the proximal section 12d of the radiopaque marker element 12. The spring 14 of the present embodiment is a compression spring, and more specifically a helical compression spring whilst in other embodiments any suitable spring may be used. In yet other embodiments, any component which applies a force on the radiopaque marker element to maintain contact with the access site may be used rather than the spring 14. The spring 14 is configured to bias the radiopaque marker element 12 towards the distal end 11b of the longitudinal sheath member 11. When the introducer sheath 10 is inserted into a body of a patient, the spring 14 will bias the radiopaque marker element 12 into the second position so that the radiopaque marker element 12 is abutting the access site (see FIG. 3).

The spring 14 eliminates the need for the physician to manually slide the radiopaque marker element 12 to the location of the access site and hold it there. This allows a more reliable and convenient determination of the location of the access site and the depth of penetration of the introducer sheath 10 into the body of the patient. The radiopaque marker element 12 may maintain abutting contact with the access site even upon movement of the longitudinal sheath member 11 to different depths in the body.

In the relaxed state (not shown), the spring 14 may be configured such that the radiopaque marker element 12 is aligned with the distal end 11b of the longitudinal sheath member 11, and specifically, the first radiopaque part 12a is aligned with the distal end 11b of the longitudinal sheath member. This allows the radiopaque marker 12 to remain abutting the access site for the full length of insertion of the longitudinal sheath member 11.

The proximal end 11c of the longitudinal sheath member 11 is connected to a sheath hub 13. The sheath hub 13 allows connection of the introducer sheath 10 to other medical devices and assists the introduction of catheters or other devices into the lumen of the longitudinal sheath member 11 and through the access site into the body of the patient.

The proximal end of the spring 14 is fixedly connected to the sheath hub 13. The spring 14 is therefore disposed between the sheath hub 13 and the radiopaque marker element 12 and biases the radiopaque marker element 12 distally away from the sheath hub 13.

The spring 14 may be made from a super-elastic material such as Nitinol, providing the resilient material properties for the spring 14. In other embodiments other elastic (i.e. resiliently deformable) materials are used for the spring, such as stainless steel.

FIG. 2 shows an access site A into the body of a patient and the introducer sheath 10 of FIG. 1 entering the body of the patient through access site A. The introducer sheath 10 is shown in a first configuration in which the radiopaque marker element 12 is at a first position along the longitudinal length of the longitudinal sheath member, where the radiopaque marker element 12 is not in contact with the access site A.

In this first position, the physician can visibly see the access site A as it is not blocked by the radiopaque marker element 12, allowing the physician to conveniently insert the longitudinal sheath member 11 into the access site A.

In order to position the radiopaque marker element 12 in the first position, the physician slides the radiopaque marker element 12 in a proximal direction along the longitudinal length of the longitudinal sheath member 11. This causes the spring 14 to compress and exert a biasing force in the distal direction onto the radiopaque marker element 12.

When the introducer sheath 10 is successfully inserted into access site A, the physician can release the radiopaque marker element 12. The biasing force exerted by the compressed spring 14 will cause the radiopaque marker element 12 to move along the longitudinal length of the longitudinal sheath member 11 towards the distal end 11b of the longitudinal sheath member 11 until it abuts the access site A. The introducer sheath 10 is now in a second configuration in which the radiopaque marker element 12 is at a second position along the longitudinal length of the longitudinal sheath member 11, which is shown in FIG. 3A and FIG. 3B.

FIG. 3A shows the introducer sheath 10 of FIG. 1 in the second configuration in which the radiopaque marker element 12 abuts the access site A such that insertion of the radiopaque marker element 12 into the access site A is prevented.

The spring 14 is still in a compressed state and will exert a force on the radiopaque marker element 12 to bias it against the body of the patient.

The distal section 12c of the radiopaque marker element 12 is larger in diameter than the access site A. The radiopaque marker element 12 therefore does not enter the body of the patient through the access site A but is biased against the body of the patient so as to abut the access site A.

Due to the larger diameter of the distal section 12c of the radiopaque marker element 12, the radiopaque marker element 12 encompasses the access site A in the second position. This allows a more accurate determination of the location of the access site A, even when the introducer sheath 10 is rotated or repositioned.

When carrying out intravascular procedures, the procedures are usually carried out under fluoroscopy to allow the physician to see the devices and surgical instruments inside the patient's body. During the course of such procedures the introducer sheath 10 may often have to be repositioned to change the depth of penetration, the angle of penetration or the angle of rotation of the introducer sheath 10.

When the introducer sheath 10 is in the second configuration, the physician can conveniently and reliably measure the distance between the radiopaque marker element 12 and the second radiopaque marker element 15 inside the body of the patient under fluoroscopy, allowing a determination of the depth of penetration of the longitudinal sheath member 11 into the body of the patient. A determination of the depth of penetration may be made, for example, by the physician measuring the distance on the screen showing the fluoroscopic images. Alternatively, an automatic measurement of distance may be made by a computer based on the fluoroscopic images which then displays the depth of penetration to the physician. By being aware of the depth of penetration of the longitudinal sheath member 11 into the body of the patient during intravascular procedures, the physician can avoid accidental sheath withdrawal.

The introducer sheath 10 allows an accurate and reliable determination of the depth of penetration of the longitudinal sheath member 11 even when the introducer sheath 10 is moved or repositioned. As long as at least part of the longitudinal sheath member 11 is disposed inside the body of the patient, the spring 14 will bias the radiopaque marker element 12 in a distal direction so that it abuts the access site A. The physician can therefore reposition the introducer sheath 10 so as to change the depth of penetration, the angle of penetration or the angle of rotation of the introducer sheath 10 while the radiopaque marker element 12 remains abutting the access site A.

FIG. 3B shows the introducer sheath 10 of FIG. 1 in the second configuration and a catheter 16 being introduced into the body of the patient through the introducer sheath 10.

Once the introducer sheath 10 is positioned in the body of the patient, the physician can use the sheath hub 13 to introduce a catheter 16 or other device into the lumen of the longitudinal sheath member 11 and through the access site A into the body of the patient. The catheter 16 is a thin flexible tube with an outer diameter smaller than the lumen of the longitudinal sheath member 11 so that it may be slidably moved inside the lumen of the longitudinal sheath member 11. The catheter 16 may be used for a number of different intravascular procedures such as drainage, administration of fluids or gases or access by surgical instruments. Surgical procedures requiring the use of a catheter 16 may include, for example, balloon angioplasty, angiography, balloon septostomy and catheter ablation.

The catheter 16 comprises a third radiopaque marker element 17 disposed in the distal section 16a of the catheter 16. The third radiopaque marker element 17 allows a physician to visualise the location of the distal section 16a of the catheter 16 under fluoroscopy whilst intravascular work is being carried out.

Furthermore, the physician can conveniently and reliably determine the distance between the third radiopaque marker 17 disposed in the distal section 16a of the catheter 16 and the second radiopaque marker element 15 disposed in the distal section 11a of the longitudinal sheath member 11, as well as the distance between the third radiopaque marker 17 disposed in the distal section 16a of the catheter 16 and the radiopaque marker element 12 abutting the access site A. In other words, the physician can conveniently and reliably determine the depth of penetration of the catheter 16 which will aid in carrying out the intravascular work as well as preventing accidental catheter withdrawal.

Various modifications will be apparent to those skilled in the art. For example, the introducer sheath 10 may not comprise a spring 14 so that the physician can manually move and hold the radiopaque marker element 12 at the access site. The spring 14 also does not have to be attached to the sheath hub 13. The spring 14 may be connected to the longitudinal sheath member 11, for example, to provide a biasing force on the radiopaque marker element.

The radiopaque marker element 12 may be comprised entirely of a first radiopaque part 12a with no non-radiopaque part.

The spring 14 may be replaced by any other biasing element such as an elastic rubber element or a magnetic biasing element.

The first radiopaque part 12a of the radiopaque marker element 12 may not be disposed directly at the distal end of the radiopaque marker element 12 but rather there may be a section of non-radiopaque material between the distal end and the first radiopaque part 12a.

Similarly, the second radiopaque marker 15 may not be disposed at the distal end 11b of the longitudinal sheath member 11 but rather disposed anywhere within the distal section 11a of the longitudinal sheath member 11.

The advantage of this is that this results in a safety margin for sheath withdrawal so that even when the radiopaque marker element 12 and the second radiopaque marker element 15 coincide there may still be part of the longitudinal sheath member 11 which is inside the body of the patient so that sheath withdrawal does not occur.

All of the above are fully within the scope of the present disclosure, and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. The invention is defined in claim 1. Preferable embodiments are defined in the dependent claims.

## Claims

1. An introducer sheath (10) for insertion into the body of a patient through an access site (A) of the body, the introducer sheath (10) comprising:
a longitudinal sheath member (11) for insertion into the access site (A) to carry out intravascular work; and
a radiopaque marker element (12) arranged over the outer surface (11d) of the longitudinal sheath member (11),
in which introducer sheath (10) the radiopaque marker element (12) is moveable along at least part of the longitudinal length of the longitudinal sheath member (11) from a first position, at which the radiopaque marker element (12) is not in contact with the access site (A), to a second position, at which the radiopaque marker element (12) abuts the access site (A) such that insertion of the radiopaque marker element (12) into the access site (A) is prevented.

2. The introducer sheath (10) of Claim 1, wherein the longitudinal sheath member (11) further comprises a second radiopaque marker element (15) fixedly disposed at a distal section (11a) thereof.

3. The introducer sheath (10) of Claim 2, wherein the second radiopaque marker element (15) is disposed at a distal end (11b) of the longitudinal sheath member (11).

4. The introducer sheath (10) of Claim 1, 2 or 3, further comprising a biasing element configured to bias the radiopaque marker element (12) to the second position.

5. The introducer sheath (10) of Claim 4, wherein the biasing element is configured such that, in the relaxed state of the biasing element, the radiopaque marker element (12) is aligned with a distal end (11b) of the longitudinal sheath member (11).

6. The introducer sheath (10) of Claim 4 or 5, wherein the biasing element is a spring (14).

7. The introducer sheath (10) of Claim 6, further comprising a sheath hub (13) disposed at a proximal end (11c) of the longitudinal sheath member (11), and wherein the spring (14) is disposed between the sheath hub (13) and the radiopaque marker element (12).

8. The introducer sheath (10) of Claim 6 or 7, wherein the spring (14) is made from Nitinol.

9. The introducer sheath (10) of any preceding claim, wherein the radiopaque marker element (12) comprises a first radiopaque part (12a) and a second non-radiopaque part (12b).

10. The introducer sheath (10) of Claim 9, wherein the first radiopaque part (12a) is disposed at a distal section (12c) of the radiopaque marker element (12).

11. The introducer sheath (10) of Claims 1 to 8, wherein the radiopaque marker element (12) consists of a radiopaque material.

12. The introducer sheath (10) of any preceding claim, wherein the radiopaque marker element (12) is tapered along a longitudinal length thereof parallel to the longitudinal axis of the longitudinal sheath member (11).

13. The introducer sheath (10) of any preceding claim, wherein a distal section (12c) of the radiopaque marker element (12) is wider than a proximal section (12d) thereof.

14. The introducer sheath (10) of any preceding claim, wherein the radiopaque marker element (12) is configured to encompass the access site (A).

15. The introducer sheath (10) of any preceding claim, wherein the radiopaque marker element (12) touches the outer surface (11d) of the longitudinal sheath member (11).

16. The introducer sheath (10) of any preceding claim, wherein the radiopaque marker element (12) is moveable along the longitudinal length of the longitudinal sheath member (11) by sliding the radiopaque marker element (12) relative to the longitudinal sheath member (11).

## Patentansprüche

1. Einführungshülse (10) zum Einführen in den Körper eines Patienten durch eine Zugangsstelle (A) des Körpers, wobei die Einführungshülse (10) Folgendes umfasst:
ein längliches Hülsenelement (11) zum Einführen in die Zugangsstelle (A), um eine intravaskuläre Arbeit durchzuführen; und
ein röntgendichtes Markierungselement (12), das über der äußeren Oberfläche (11d) des länglichen Hülsenelements (11) angeordnet ist,
wobei in der Einführungshülse (10) das röntgendichte Markierungselement (12) entlang zumindest eines Teils der Längslänge des länglichen Hülsenelements (11) von einer ersten Position, in der das röntgendichte Markierungselement (12) nicht mit der Zugangsstelle (A) in Kontakt steht, in eine zweite Position, in der das röntgendichte Markierungselement (12) an der Zugangsstelle (A) anliegt, beweglich ist, so dass ein Einführen des röntgendichten Markierungselements (12) in die Zugangsstelle (A) verhindert wird.

2. Einführungshülse (10) nach Anspruch 1, wobei das längliche Hülsenelement (11) weiter ein zweites röntgendichtes Markierungselement (15) umfasst, das fest an einem distalen Abschnitt (11a) davon angeordnet ist.

3. Einführungshülse (10) nach Anspruch 2, wobei das zweite röntgendichte Markierungselement (15) an einem distalen Ende (11b) des länglichen Hülsenelements (11) angeordnet ist.

4. Einführungshülse (10) nach Anspruch 1, 2 oder 3, weiter umfassend ein Vorspannelement, das dazu konfiguriert ist, das röntgendichte Markierungselement (12) in die zweite Position vorzuspannen.

5. Einführungshülse (10) nach Anspruch 4, wobei das Vorspannelement so konfiguriert ist, dass im entspannten Zustand des Vorspannelements das röntgendichte Markierungselement (12) mit einem distalen Ende (11b) des länglichen Hülsenelements (11) ausgerichtet ist.

6. Einführungshülse (10) nach Anspruch 4 oder 5, wobei das Vorspannelement eine Feder (14) ist.

7. Einführungshülse (10) nach Anspruch 6, weiter umfassend eine Hülsennabe (13), die an einem proximalen Ende (11c) des länglichen Hülsenelements (11) angeordnet ist, und wobei die Feder (14) zwischen der Hülsennabe (13) und dem röntgendichten Markierungselement (12) angeordnet ist.

8. Einführungshülse (10) nach Anspruch 6 oder 7, wobei die Feder (14) aus Nitinol hergestellt ist.

9. Einführungshülse (10) nach einem vorstehenden Anspruch, wobei das röntgendichte Markierungselement (12) einen ersten röntgendichten Teil (12a) und einen zweiten nicht röntgendichten Teil (12b) umfasst.

10. Einführungshülse (10) nach Anspruch 9, wobei der erste röntgendichte Teil (12a) an einem distalen Abschnitt (12c) des röntgendichten Markierungselements (12) angeordnet ist.

11. Einführungshülse (10) nach den Ansprüchen 1 bis 8, wobei das röntgendichte Markierungselement (12) aus einem röntgendichten Material besteht.

12. Einführungshülse (10) nach einem vorstehenden Anspruch, wobei das röntgendichte Markierungselement (12) entlang einer Längslänge davon parallel zur Längsachse des länglichen Hülsenelements (11) verjüngt ist.

13. Einführungshülse (10) nach einem vorstehenden Anspruch, wobei ein distaler Abschnitt (12c) des röntgendichten Markierungselements (12) breiter ist als ein proximaler Abschnitt (12d) davon.

14. Einführungshülse (10) nach einem vorstehenden Anspruch, wobei das röntgendichte Markierungselement (12) so konfiguriert ist, dass es die Zugangsstelle (A) umschließt.

15. Einführungshülse (10) nach einem vorstehenden Anspruch, wobei das röntgendichte Markierungselement (12) die äußere Oberfläche (11d) des länglichen Hülsenelements (11) berührt.

16. Einführungshülse (10) nach einem vorstehenden Anspruch, wobei das röntgendichte Markierungselement (12) entlang der Längslänge des länglichen Hülsenelements (11) beweglich ist, indem das röntgendichte Markierungselement (12) relativ zum länglichen Hülsenelement (11) verschoben wird.

## Revendications

1. Gaine d'introduction (10) pour une insertion dans le corps d'un patient à travers un site d'accès (A) du corps, la gaine d'introduction (10) comprenant :
un élément de gaine longitudinal (11) pour une insertion dans le site d'accès (A) pour réaliser un travail intravasculaire ; et
un élément marqueur radio-opaque (12) agencé sur la surface extérieure (11d) de l'élément de gaine longitudinal (11),
dans laquelle gaine d'introduction (10) l'élément marqueur radio-opaque (12) est mobile le long d'au moins une partie de la longueur longitudinale de l'élément de gaine longitudinal (11) à partir d'une première position, au niveau de laquelle l'élément marqueur radio-opaque (12) n'est pas en contact avec le site d'accès (A), jusqu'à une seconde position, au niveau de laquelle l'élément marqueur radio-opaque (12) vient en butée contre le site d'accès (A) de sorte qu'une insertion de l'élément marqueur radio-opaque (12) dans le site d'accès (A) soit empêchée.

2. Gaine d'introduction (10) selon la revendication 1, dans laquelle l'élément de gaine longitudinal (11) comprend en outre un second élément marqueur radio-opaque (15) disposé de manière fixe au niveau d'une section distale (11a) de celui-ci.

3. Gaine d'introduction (10) selon la revendication 2, dans laquelle le second élément marqueur radio-opaque (15) est disposé à une extrémité distale (11b) de l'élément de gaine longitudinal (11).

4. Gaine d'introduction (10) selon la revendication 1, 2 ou 3, comprenant en outre un élément de sollicitation configuré pour solliciter l'élément marqueur radio-opaque (12) vers la seconde position.

5. Gaine d'introduction (10) selon la revendication 4, dans laquelle l'élément de sollicitation est configuré de sorte que, dans l'état relâché de l'élément de sollicitation, l'élément marqueur radio-opaque (12) soit aligné avec une extrémité distale (11b) de l'élément de gaine longitudinal (11).

6. Gaine d'introduction (10) selon la revendication 4 ou 5, dans laquelle l'élément de sollicitation est un ressort (14).

7. Gaine d'introduction (10) selon la revendication 6, comprenant en outre un moyeu de gaine (13) disposé à une extrémité proximale (11c) de l'élément de gaine longitudinal (11), et dans laquelle le ressort (14) est disposé entre le moyeu de gaine (13) et l'élément marqueur radio-opaque (12).

8. Gaine d'introduction (10) selon la revendication 6 ou 7, dans laquelle le ressort (14) est fabriqué à partir de Nitinol.

9. Gaine d'introduction (10) selon une quelconque revendication précédente, dans laquelle l'élément marqueur radio-opaque (12) comprend une première partie radio-opaque (12a) et une seconde partie radio-opaque (12b).

10. Gaine d'introduction (10) selon la revendication 9, dans laquelle la première partie radio-opaque (12a) est disposée au niveau d'une section distale (12c) de l'élément marqueur radio-opaque (12).

11. Gaine d'introduction (10) selon les revendications 1 à 8, dans laquelle l'élément marqueur radio-opaque (12) consiste en un matériau radio-opaque.

12. Gaine d'introduction (10) selon une quelconque revendication précédente, dans laquelle l'élément marqueur radio-opaque (12) est conique le long d'une longueur longitudinale de celui-ci parallèle à l'axe longitudinal de l'élément de gaine longitudinal (11).

13. Gaine d'introduction (10) selon une quelconque revendication précédente, dans laquelle une section distale (12c) de l'élément marqueur radio-opaque (12) est plus large qu'une section proximale (12d) de celui-ci.

14. Gaine d'introduction (10) selon une quelconque revendication précédente, dans laquelle l'élément marqueur radio-opaque (12) est configuré pour englober le site d'accès (A).

15. Gaine d'introduction (10) selon une quelconque revendication précédente, dans laquelle l'élément marqueur radio-opaque (12) touche la surface extérieure (11d) de l'élément de gaine longitudinal (11).

16. Gaine d'introduction (10) selon une quelconque revendication précédente, dans laquelle l'élément marqueur radio-opaque (12) est mobile le long de la longueur longitudinale de l'élément de gaine longitudinal (11) par coulissement de l'élément marqueur radio-opaque (12) relativement à l'élément de gaine longitudinal (11).
